# EUROPEAN PATENT APPLICATION

(11) **EP 1 582 185 A1**
(43) Date of publication of application: **05.10.2005**
(21) Application number: 03777312.4
(22) Date of filing: 05.12.2003
(51) Int. Cl.: A61F 5/453, A61F 5/44, A61F 13/15

(54) **DISPOSABLE DIAPER**

(30) Priority: 17.12.2002 JP 2002365574
(71) Applicant: UNI-CHARM CO., LTD., Ehime 799-0111 (JP)
(72) Inventor: SUGITO, Tomoko Technical Center, UNI-CHARM CO LTD, Mitoyo-gun Kagawa 769-1602 (JP)
(74) Representative: Sperling, Rüdiger
(86) International application number: PCT/JP2003/015625
(87) International publication number: WO 2004/054483

(57) **Abstract**

A diaper (1A) includes a liquid-absorbent core (2) extending between front and rear waist regions (5, 7), a liquid-pervious first sheet covering upper and lower surfaces (11, 12) of the core and a liquid-impervious second sheet (4). The diaper (1A) is formed with a cleaved zone (13) continuously extending in the front waist region (5) and the crotch region (6) in a longitudinal direction of the diaper (1A) and extending through the core (2). Along the cleaved zone (13), an upper covering section (14) of the first sheet (3) extends downward from the upper surface (11) of the core (2) and a lower covering section (15) of the first sheet (3) also extends downward along the cleaved zone (13). The upper and lower covering sections (14, 15) extend outward in a transverse direction of the diaper (1A) symmetrically about the cleaved zone (13) to define a penis pocket (16) below the cleaved zone (13).

## Description

### TECHNICAL FIELD OF THE INVENTION

The present invention relates to disposable diapers for absorption and containment of bodily discharges.

### BACKGROUND ART OF THE INVENTION

There is disclosed Japanese Patent Publication No. 1994-178788A disposable diapers, comprising a liquid-pervious first sheet facing a wearer's body, a liquid-impervious sheet opposite to the first sheet, a liquid-absorbent core interposed between the sheets and a pair of flap members attached to an upper surface of the first sheet and extending in a longitudinal direction. The flap members respectively have fixed lateral zones so as to overlie the core and free zones let free from the first sheet. The free zones extend inward from the respective fixed lateral zones in a transverse direction of the diaper.

In the case of the diaper disclosed in the above-cited Publication, a distance between the fixed lateral zones of the respective flap members as well as a length from the fixed lateral zone to the associated free zone are dimensioned so that a wearer's penis can be held between the free zones of the flap members. According to the disclosure of the above-cited Publication, a wearer's penis can be placed on the core and thereby discharged urine can be reliably absorbed by the core because a wearer's penis is held between the free zones of the flap members.

While a material for the flap members is not specified in the disclosure of the above-cited Publication, the flap members have usually been formed from a plastic film or a sheet such as a fibrous nonwoven fabric. If the flap members are formed from the plastic film or the fibrous nonwoven fabric, the flap members will be flexible and readily deformed and therefore it will be difficult to hold a wearer's penis on the core. Specifically, even if a wearer's penis is put between the free zones of the flap members when the diaper disclosed in the above-cited Publication is put on a wearer's body, there is an anxiety that the penis might get out of the space defined between the free zones due to the movement of the wearer and it is difficult to reliably hold the penis at a predetermined position of the diaper.

It is an object of the present invention to provide a disposable diaper improved so that a wearer's penis can be held at a predetermined position without a fear that the penis might shift from this predetermined position.

### DISCLOSURE OF THE INVENTION

According to the present invention, there is provided a disposable diaper having a longitudinal direction, a transverse direction, a front waist region, a rear waist region and a crotch region extending between the front and rear waist regions comprising: a liquid-absorbent core having a body facing upper surface and a lower surface opposite to the upper surface and extending between the front and rear waist regions, a liquid-pervious first sheet having an upper covering section adapted to cover said upper surface of the core and a lower covering section adapted to cover the lower surface of the core and a liquid-impervious second sheet lying outside the lower covering section of the first sheet.

The present invention further comprises the core being provided with a cleaved zone extending in the longitudinal direction and extending through a thickness thereof and the upper covering section of the first sheet continuously extending downward from the upper surface of the core along the cleaved zone and further extending outward in the transverse direction so as to define a penis pocket extending in the longitudinal direction below the cleaved zone.

The present invention includes the following embodiments.

The lower covering section of the first sheet extends downward along the cleaved zone of the core and further extends outward in the transverse direction so that the lower covering section cooperates with the upper covering section to define the penis pocket.

The lower surface of the core is let free from the lower covering section of the first sheet and the lower covering section is joining to the second sheet along a peripheral edge of the core so that the penis pocket is movable below said cleaved zone of the core.

Except the cleaved zone of the core, the upper covering section of the first sheet has an outer surface of the core covered with a liquid-impervious third sheet.

Of the front and rear waist regions and the crotch region, at least the front waist region is formed in a transversely middle zone thereof with the cleaved zone of the core.

### BRIEF DESCRIPTION OF THE DRAWINGS

Fig. 1 is a partially cut away perspective view of a diaper as an embodiment of the present invention;
Fig. 2 is a sectional view taken along line II-II in Fig. 1;
Fig. 3 is a sectional view taken along line III-III in Fig. 1;
Fig. 4 is a view similar to Fig. 2, depicting the diaper as a wearer's penis put in a penis pocket;
Fig. 5 is a partially cutaway perspective view showing a diaper as another embodiment of the present invention;
Fig. 6 is a sectional view taken along line VI-VI in Fig. 5; and
Fig. 7 is a sectional view taken along line VII-VII in Fig. 5.

### DESCRIPTION OF THE BEST MODE FOR WORKING OF THE INVENTION

Details of a disposable diaper according to the present invention will be more fully understood from the description given hereunder with reference to the accompanying drawings.

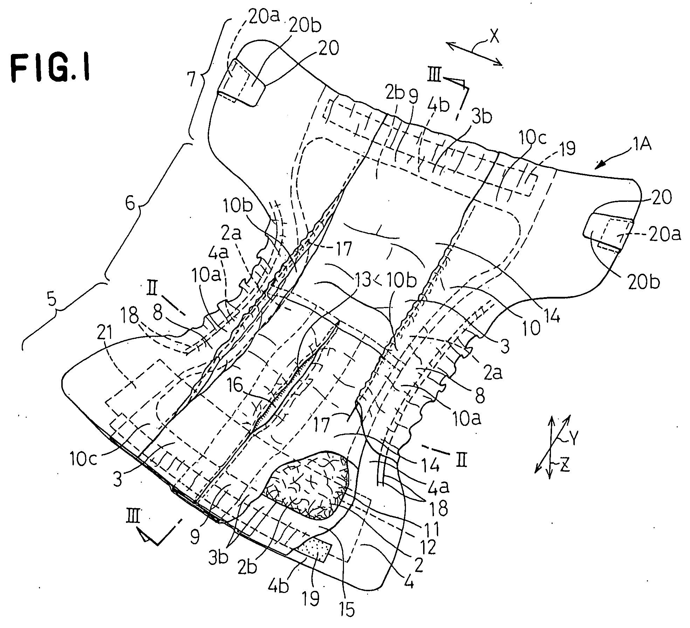

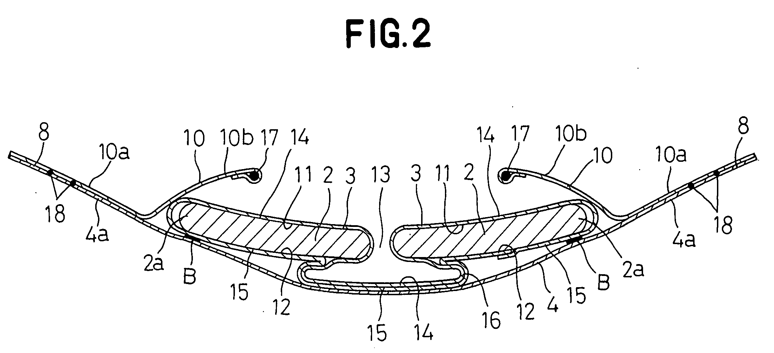

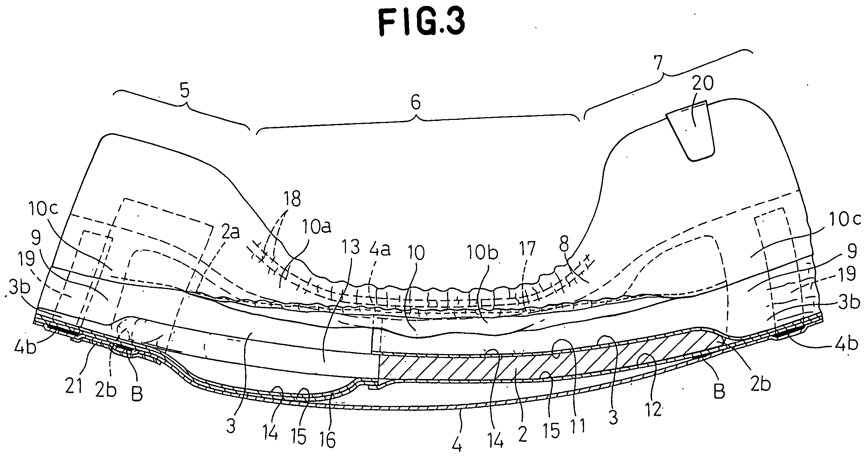

Fig. 1 is a partially cutaway perspective view showing a diaper 1A as an embodiment of the present invention, Fig. 2 is a sectional view taken along line II-II in Fig. 1 and Fig. 3 is a sectional view taken along line III-III in Fig. 1. In Fig. 1, a transverse direction is indicated by an arrow X, a longitudinal direction is indicated by an arrow Y and a thickness direction is indicated by an arrow Z. Expression "inner surfaces" of first and second sheets 3, 4 and a leak-proof sheet 10 refers to the respective surfaces facing a core 2 and expression "outer surfaces" of these sheets 3, 4, 10 refers to the respective surfaces facing away from the core 2.

A diaper 1A comprises the liquid-absorbent core 2, the liquid-pervious first sheet 3 and the liquid-impervious second sheet 4 extending outside a lower covering section 15 of the first sheet 3. The diaper 1A is composed of, as viewed in the longitudinal direction, a front waist region 5, a rear waist region 7 and a crotch region 6 extending between the waist regions 5, 7. The diaper 1A is of the so-called open type adapted to be put on a wearer's body with the front and rear waist regions 5, 7 connected with each other.

The diaper 1A has a pair of side flaps 8 extending in the longitudinal direction outside transversely side edges 2a (peripheral edges) of the core 2 and a pair of end flaps 9 extending in the transverse direction outside longitudinally opposite ends 2b (peripheral edges). In the crotch region 6, the side flaps 8 respectively describe circular arcs which are convex inward in the transverse direction of the diaper 1A. The diaper 1A has a generally hourglass-like planar shape. A pair of liquid-impervious leak-barrier flaps 10 are attached to the respective side flaps 8 so as to extend in the longitudinal direction.

The core 2 comprises a mixture of fluff pulp fibers and super-absorbent polymer particles or a mixture of fluff pulp, super-absorbent polymer particles and thermoplastic synthetic resin fibers, in any case, compressed to a desired thickness. This means that the core 2 has a stiffness higher than those of the first sheet 3, the second sheet 4 and the leak-barrier flaps 10. Preferably, the core 2 is entirely wrapped with a liquid-pervious sheet such as tissue paper in order to prevent the core 2 from get out of its initial shape and to the polymer particles from falling off.

The core 2 has a body facing upper surface 11 and a lower surface 12 opposite to the upper surface 11, and extends between the front waist region 5 and the rear waist region 7. The core 2 has a cleaved zone 13 extending through the core 2 in the thickness direction between the upper and lower surfaces 11, 12 and extending in the longitudinal direction. The cleaved zone 13 is adapted to be opened and closed in the transverse direction. The cleaved zone 13 is formed in transversely middle zones of the front waist region 5 and the crotch region 6 so that the cleaved zone 13 extends partially in the front waist region 5 and further into a generally front half of the crotch region 6. In other words, the cleaved zone 13 is formed in a transversely middle zone of the core 2 and extends in the longitudinal direction over approximately a front half of the core 2.

The first sheet 3 has an upper covering section 14 adapted to cover the entire upper surface 11 of the core 2 and a lower covering section 15 adapted to cover the entire lower surface 12 of the core 2. The upper covering section 14 continuously extends downward from the upper surface 11 of the core 2 along the cleaved zone 13 and then extends outward in the transverse direction symmetrically about the cleaved zone 13 so as to be exposed below and through the cleaved zone 13. The lower covering section 15 of the first sheet 3 extends downward beyond the cleaved zone 13 and further extends outward in the transverse direction between the core 2 and the second sheet 4. Of the first sheet 3, the upper covering section 14 cooperates with the lower covering section 15 to define a penis pocket 16 below the cleaved zone 13. The pocket 16 has a generally inverted Ω-shaped cross-section and extends along the cleaved zone 13 in the longitudinal direction (See Figs. 2 and 3).

The upper covering section 14 has its inner surface not joined to the upper surface 11 of the core 2, i.e., let free from the upper surface 11. The lower covering section 15 has its outer surface joined along the transversely opposite side edges 2a (peripheral edge) and the longitudinally opposite ends 2b (peripheral edge) to the inner surface of the second sheet 4 by means of an adhesive B. In the remaining region of the core 2 except the opposite side edges 2a and opposite ends 2b, the lower covering section 15 is not joined to the second sheet 4 i.e. , let free from the second sheet 4. While the penis pocket 16 has its bottom in contact with the second sheet 4, the bottom of the pocket 16 is not joined to the second sheet 4, i.e., is let free from the second sheet 4. Thus the pocket 16 is freely movable below the cleaved zone 13. It should be understood that the upper covering section 14 and the lower covering section 15 may either be joined together or be not joined together.

The leak-barrier flaps 10 extend in the longitudinal direction between the end flaps 9. Each of the leak-barrier flaps 10 has a fixed lateral zone 10a extending outside the associated side edge 2a of the core in the longitudinal direction, a movable zone 10b extending in the longitudinal direction and normally biased to rise on the upper covering section 14 of the first sheet 3 and longitudinally opposite fixed end zones 10c lying in the front and rear waist regions 5, 7 and collapsed inward in the transverse direction of the diaper 1A. The movable zone 10b is provided along its upper edge with a stretchable elastic member 17 extending in the longitudinal direction. The elastic member 17 is contractibly attached to the movable zone 10b and covered with a part of the movable zone 10b.

The side flaps 8 are formed from the lateral marginal zones 4a of the second sheet 4 extending outward beyond the side edges 2a of the core 2 in the transverse direction and the fixed lateral zones 10a of the leak-barrier flap 10. In each of the side flaps 8, the lateral marginal zone 4a of the second sheet 4 is overlapped together with the fixed lateral zone 10a of the leak-barrier flap 10 and, the lateral marginal zone 4a and the fixed lateral zone 10a having their inner surfaces joined to each other. The side flap 8 is provided with a plurality of leg elastic members 18 extending in the longitudinal direction and contractibly attached thereto. The leg elastic members 18 are interposed between the lateral marginal zone 4a of the second sheet 4 and the fixed lateral zone 10a of the leak-barrier flap 10 and joined to the inner surfaces of the sheets 4, 10.

The end flaps 9 are formed from longitudinally opposite end zones 3b of the first sheet 3 and longitudinally opposite end zones 4b of the second sheet 4. In each of the end flaps 9, the end zones 3b, 4b of the first and second sheets 3, 4 are overlapped together and the sheets 3, 4 have their inner surfaces bonded to each other along the end zones 3b, 4b. In each of the end flaps 9, the upper covering section 14 and the lower covering section 15 of the first sheet cooperating to form the pocket 16 are folded back and joined in this state to the end zones 4b of the second sheet 4. The fixed end zones 10c of the leak-barrier flaps 10 are bonded to the outer surface of the end zone 3b of the first sheet 3. The end flap 9 is provided with a ribbon-like waist elastic member 19 extending in the transverse direction and contractibly attached thereto. The waist elastic member 19 is interposed between the end zone 3b of the first sheet and the end zone 4b of the second sheet 4 and attached to the inner surfaces of the sheets 3, 4.

In the rear waist region 7, the side flaps 8 are respectively provided with flexible tape fasteners 20 made of a plastic film. Each of the tape fasteners 20 has a fixed end zone 20a and a free end zone 20b both extending in the transverse direction. The fixed end zone 20a is interposed between the lateral marginal zone 4b of the second sheet 4 and the fixed lateral zone 10b of the leak-barrier flap 10 and joined to the inner surfaces of the sheets 4, 10. The inner surface of the free end zone 20b is coated with a pressure-sensitive adhesive (not shown). The free end zone 20b is folded inward in the transverse direction and temporarily joined to the lateral zone 10b of the leak-barrier flap 10 by means of a pressure-sensitive adhesive before the diaper 1A is actually used. The front waist region 5 is provided with a flexible target tape strip 21 on which the free end zone 20b of the tape fastener 20 is detachably anchored. The target tape strip 21 is made of a plastic film and shaped in a rectangle of which the longer sides extend in the transverse direction. The target tape strip 21 is joined to the outer surface of the second sheet 4.

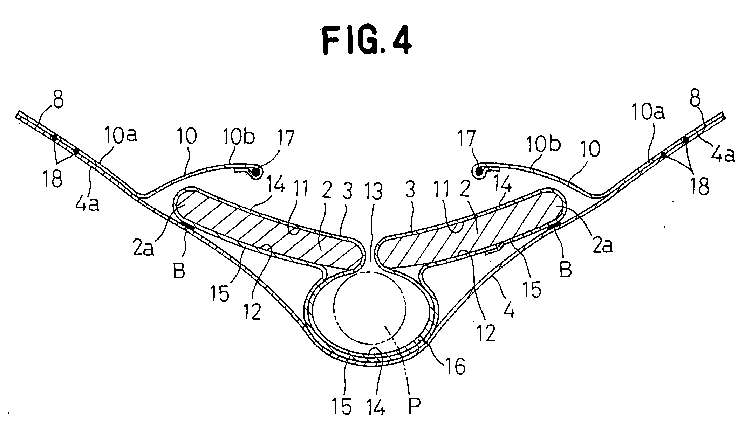

Fig. 4 is a view similar to Fig. 2, depicting the diaper 1A as a wearer's penis P put in the pocket 16. To put the diaper 1A on a wearer's body, the cleaved zone 13 of the core 2 is opened outward in the transverse direction and then a wearer's penis P is put into the pocket 16. Then the side flaps 8 in the rear waist region 7 are overlapped on the outer side of the side flaps 8 in the front waist region 5 and the free end zones 20b of the respective tape fasteners 20 are anchored on the target tape strip 21 by means of a pressure-sensitive adhesive to connect the front waist region 5 and the rear waist region 7 with each other. Thereupon the diaper 1A is formed with a waist-hole and a pair of leg-holes lying below the waist-hole (not shown).

A wearer's penis P is put in the pocket 16 defined by the upper covering section 14 and the lower covering section 15 and the cleaved zone 13 of the core 2 having been opened in the transverse direction is closed again to hold the wearer's penis P. In this way, the wearer's penis P is stabilized in a transversely middle zone of the front waist region 5.

The core 2 has a stiffness higher than those of the sheets 3, 4, 10 and correspondingly higher resistance to deformation than the sheets 3, 4, 10, so the wearer's penis P is reliably held by the lower surface 12 of the core 2 and the cleaved zone 13 without anxiety that the cleaved zone 13 of the core 2 might be unintentionally opened. Thus it is unlikely that the penis P might creep out from the pocket 16 even if the movement of a wearer's body is transmitted to the diaper 1A.

Urine discharged on the diaper 1A put on a wearer's body is permeated through the pocket 16 to the lower surface 12 of the core 2, then absorbed and contained by the core 2. The core 2 lying above the pocket 16 serves also as barrier against urine, which reliably prevents the amount of urine discharged within the pocket 16 from seeping through the upper covering section 14 of the first sheet 3.

The elastic members 17 contract and thereby cause the movable zones 10b of the respective leak-barrier flaps 10 to rise on the upper covering section 14 as the diaper 1A curves in the longitudinal direction with the upper covering section 14 of the first sheet 3 inside. Even if, during use of the diaper 1A, an amount of urine is discharged on the outer surface of the upper covering section 14 of the first sheet 3, the movable zones 10b of the respective leak-barrier flaps 10 rising above the upper covering section 14 function as the barrier against urine and prevent any amount of urine from leaking sideways beyond the side flaps 8.

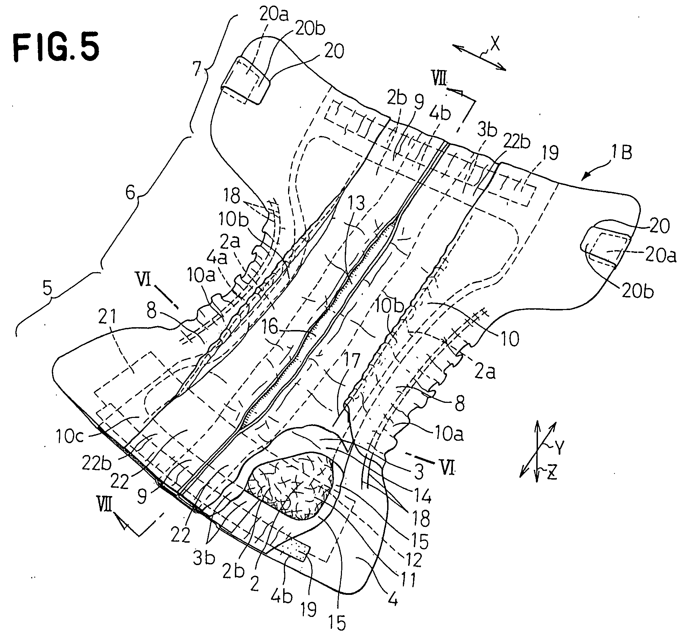

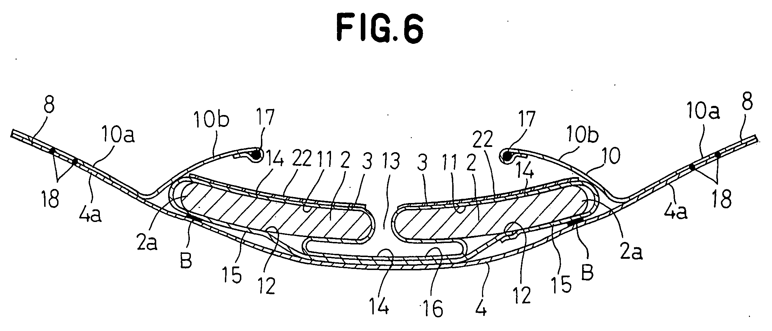

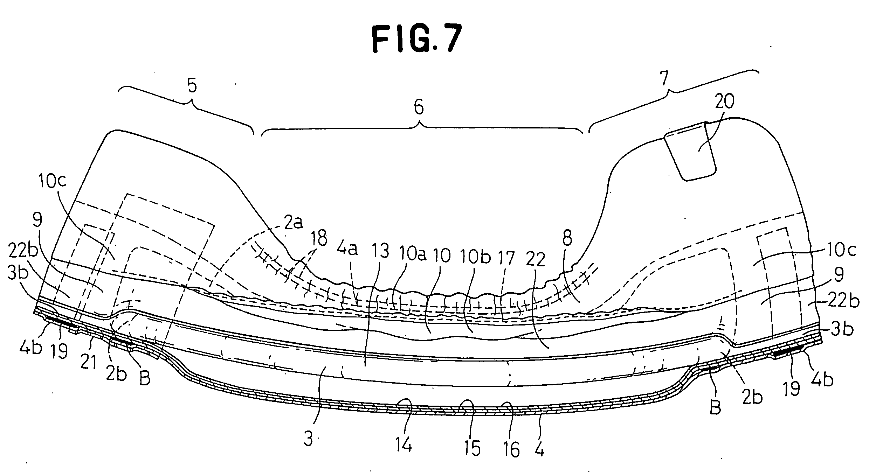

Fig. 5 is a partially cutaway perspective view of a diaper 1B as another embodiment of the present invention, Fig. 6 is a sectional view taken along line VI-VI in Fig. 5 and Fig. 7 is a sectional view taken along line VII-VII in Fig. 5. In Fig. 5, a transverse direction is indicated by an arrow X, a longitudinal direction is indicated by an arrow Y and a thickness direction is indicated by an arrow Z. Expression "outer surfaces" of a third sheet 22 refers to its surface facing a core 2 and expression "inner surface" of the third sheet 22 refers to its surface facing away from the core 2.

The diaper 1B comprises the liquid-absorbent core 2, the liquid-pervious first sheet 3, the liquid-impervious second sheet 4 and a liquid-impervious third sheet 22. The diaper 1B is composed of a front waist region 5, a rear waist region 7, a crotch region 6 extending between the waist regions 5, 7, a pair of side flaps 8 extending in the longitudinal direction and a pair of end flaps 9 extending in the transverse direction.

Similar to the case shown by Fig. 1, the core 2 extends between the front and rear waist regions 5, 7. The core 2 has an upper surface 11 facing a wearer's body and a lower surface 12 opposite to the upper surface 11. The core 2 extends between the front waist region 5 and the rear waist region 7. The core 2 has a cleaved zone 13 extending through the core 2 in the thickness direction between the upper and lower surfaces 11, 12 and extending in the longitudinal direction. The cleaved zone 13 is adapted to be opened and closed in the transverse direction. The cleaved zone 13 extends between the front and rear waist regions 5, 7 and is continuously formed in a transversely middle zone of the regions 5, 6, 7. In other words, the cleaved zone 13 is formed in a transversely middle zone of the core 2 and extends in the longitudinal direction over a full length of the core 2.

The first sheet 3 has an upper covering section 14 adapted to cover the entire upper surface 11 of the core 2 and a lower covering section 15 adapted to cover the entire lower surface 12 of the core 2. The upper covering section 14 continuously extends downward from the upper surface 11 of the core 2 along the cleaved zone 13 and then extends outward in the transverse direction symmetrically about the cleaved zone 13 so as to be exposed below and through the cleaved zone 13. Of the first sheet 3, the upper covering section 14 defines a penis pocket 16 below the cleaved zone 13. The pocket 16 has a generally inverted Ω-shaped cross-section and extends along the cleaved zone 13 in the longitudinal direction (See Figs. 6 and 7).

The third sheet 22 covers the entire outer surface of the upper covering section 14 of the first sheet 3 except for the cleaved zone 13 of the core 2. The third sheet 22 has its inner surface bonded to the outer surface of the upper covering section 14.

The upper covering section 14 has its inner surface not joined to the upper surface 11 of the core 2, i.e., let free from the core 2. The lower covering section 15 has its inner surface not joined to the lower surface 12 of the core 2, i.e., let free from the core 2. The lower covering section 15 has its outer surface bonded to the inner surface of the second sheet 4 along the transversely opposite side edges 2a (peripheral edge) and the longitudinally opposite ends 2b (peripheral edge) by means of an adhesive B. In the remaining region of the core 2 except the opposite side edges 2a and opposite ends 2b, the lower covering section 15 is not joined to the second sheet 4 i.e. , let free from the second sheet 4. While the penis pocket 16 has its bottom in contact with the second sheet 4, the bottom of this pocket 16 is not joined to the second sheet 4, i.e., is let free from the second sheet 4. Thus the pocket 16 is freely movable below the cleaved zone 13.

Each of the leak-barrier flaps 10 has a fixed lateral zone 10a extending in the longitudinal direction, a movable zone 10b normally biased to rise on the upper covering section 14 of the first sheet 3 and longitudinally opposite fixed end zones 10c lying in the front and rear waist regions 5, 7 and collapsed inward in the transverse direction of the diaper 1B. The movable zone 10b is provided along its upper edge with stretchable elastic member 17 extending in the longitudinal direction and contractibly attached thereto.

The side flaps 8 are formed from the lateral marginal zones 4a of the second sheet 4 extending outward beyond the side edges 2a of the core 2 in the transverse direction and the fixed lateral zones 10a of the leak-barrier flaps 10. In each of the side flaps 8, the lateral marginal zone 4a is overlapped together with the fixed lateral zone 10a and, the lateral marginal zone 4a and the fixed lateral zone 10a having their inner surfaces joined to each other. The side flap 8 is provided with a plurality of leg elastic members 18 extending in the longitudinal direction and contractibly attached thereto. The leg elastic members 18 are interposed between the lateral marginal zone 4a of the second sheet 4 and the fixed lateral zone 10a of the leak-barrier flap 10 and bonded to the inner surfaces of the sheets 4, 10.

The end flaps 9 are formed from the end zones 3b, 4b, 22b of the first, second and third sheets 3, 4, 22 extending outward beyond the longitudinally opposite ends 2b of the core 2. In each of the end flaps 9, the end zones 3b, 4b, 22b of the first, second and third sheets 3, 4, 22 are overlapped together and, along the end zones 3, 4, 22, the first and second sheets 3, 4 have their inner surfaces bonded to each other while the inner surface of the second sheet 4 and the outer surface of the third sheets 22 are joined to each other. In each of the end flaps 9, the upper covering section 14 of the first sheet 3 constituting the pocket 16 is folded back and joined in this state to the lower covering section 15. The fixed end zones 10c of the leak-barrier flaps 10 are joined to the outer surface of the end zone 22b of the third sheet 22. The end flap 9 is provided with a ribbon-like waist elastic member 19 extending in the transverse direction and contractibly attached thereto. The waist elastic member 19 is attached to the inner surfaces of the first sheet 3 and the second sheet 4.

In the rear waist region 7, the side flaps 8 are respectively provided with tape fasteners 20 having free end zones 20b coated with a pressure-sensitive adhesive. The front waist region 5 is provided with a target tape strip 21 shaped in a rectangle of which the longer sides extend in the transverse direction and on which the free end zone 20b of the tape fastener 20 is detachably anchored. The tape fasteners 20 and the target tape strip 21 are made of a plastic film.

To put the diaper 1B on a wearer's body, the cleaved zone 13 of the core 2 is opened outward in the transverse direction and then a wearer's penis P is put into the pocket 16. Then the side flaps 8 in the rear waist region 7 are overlapped on the outer side of the side flaps 8 in the front waist region 5 and the free end zones 20b of the respective tape fasteners 20 are anchored on the target tape strip 21 by means of pressure-sensitive adhesive.

The wearer's penis P is put in the pocket 16 defined by the upper covering section 14 of the first sheet 3 and the cleaved zone 13 of the core 2 having been opened in the transverse direction is closed again to hold the wearer's penis P (See Fig. 4). In this way, the wearer's penis P is stabilized in a transversely middle zone of the front waist region 5.

The core 2 has relatively high a stiffness and correspondingly higher resistance, so the wearer's penis P is reliably held by the lower surface 12 of the core 2 and the cleaved zone 13 without anxiety that the cleaved zone 13 of the core 2 might be unintentionally opened. Thus it is unlikely that the penis P might creep out from the pocket 16.

Urine discharged on the diaper 1B put on a wearer's body is permeated through the pocket 16 to the lower surface 12 of the core 2, then absorbed and contained by the core 2. In the case of the diaper 1B also, the core 2 lies above the pocket 16 and reliably prevents the amount of urine discharged within the pocket 16 from seeping through the upper covering section 14 of the first sheet 3. In addition, the outer surface of the upper covering section 14 is covered with the liquid-impervious third sheet 22. Consequently, even if a certain amount of urine seep through the upper covering section 14, the presence of this third sheet 22 reliably prevents any amount of urine from exuding to the inner side of the diaper 1B.

In the case of this diaper 1B also, even if, during use of the diaper 1B, an amount of urine is discharged on the outer surface of the third sheet 22, the movable zones 10b of the respective leak-barrier flaps 10 rising above the upper covering section 14 function as the barrier against urine and prevent any amount of urine from leaking sideways beyond the side flaps 8.

The liquid-pervious first sheet 3 may be formed from a hydrophilic fibrous nonwoven fabric. A stock material for the liquid-impervious second sheet 4, the liquid-impervious leak-proof sheets 10 and the liquid-impervious third sheet 22 may be selected from the group consisting of a hydrophobic fibrous nonwoven fabric, a breathable liquid-impervious plastic film, a composite sheet comprising two or more layers of hydrophobic fibrous nonwoven fabrics laminated one upon another, and a composite sheet comprising a hydrophobic fibrous nonwoven fabric and a breathable liquid-impervious plastic film. It may be used to a composite nonwoven fabric comprising melt blown fibrous nonwoven fabric having a high water-resistance and a spun bond fibrous nonwoven fabric exhibiting high strength and flexibility laminated on at least one surface of the melt blown fibrous nonwoven fabric (SM nonwoven fabric or SMS nonwoven fabric) to form the second sheet 4, the leak-barrier flaps 10 and the third sheet 22.

The fibrous nonwoven fabric used for exploitation of the present invention may be selected from the group consisting of those obtained by spun lacing-, needle punching-, melt blowing-, thermal bonding-, spun bonding-, chemical bonding- and air through-processes, respectively. The component fibers of the nonwoven fabric may be selected from the group consisting of polyolefin-, polyester- and polyamide-based fibers and core-sheath-type or side-by-side-type conjugate fibers of polyethylene/polypropylene or polyethylene/polyester.

Joining of the first and second sheets 3, 4 to each other, bonding of the first and third sheets 3, 22 to each other, joining of the leak-barrier flaps to the sheets 3, 4, 22 and joining of the respective elastic members 17, 18, 19 to the sheets 3, 4, 10 may be achieved by use of an adhesive or welding techniques such as heat-sealing or sonic sealing.

The adhesive may be selected from the group consisting of a hot met adhesive, an acrylic adhesive and an elastomeric adhesive. A coating pattern for the adhesive may be selected from the group consisting of a spiral pattern, a zigzag pattern, a dot pattern and a striped pattern. By coating the sheets 3, 4, 22 in such patterns, the adhesive-coated region and the adhesive-free region are formed so that the sheets 3, 4, 10, 22 may be intermittently bonded one to another by means of the adhesive.

In the diaper 1A of Fig. 1, it is possible to cover the outer surface of the upper covering section 14 of the liquid-pervious first sheet 3 except for the cleaved zone 13 of the core 2 with the liquid-impervious third sheet 22. It is also possible for this diaper 1A to define the pocket 16 by the upper covering section 14 of the first sheet 3 alone in the same manner as in the case of Fig. 5. It is possible for the diaper 1B of Fig. 5, in turn, to define the pocket 16 by the upper covering section 14 and the lower covering section 15 of the first sheet 3 in the same manner as in the case of Fig. 1.

The present invention is applicable not only to the open-type diaper 1A, 1B of which the front and rear waist regions are connected with each other immediately before the diaper 1A, 1B is put on a wearer's body but also to the pants-type diaper of which the front and rear waist regions are previously connected with each other to form the waist-hole and the leg-holes.

The disposable diaper according to the present invention allows a wearer's penis to be held by the cleaved zone of the core and thereby to be stabilized at a predetermined position as a wearer's penis is put in the pocket formed below the cleaved zone of the core. The core has sufficiently stiff to resist an undesirable deformation and therefore a wearer's penis is reliably held by the lower surface and cleaved zone of the core without the anxiety that the cleaved zone of the core might be unintentionally opened. With the diaper according to the invention, a wearer's penis can be reliably stabilized at the predetermined position without the possibility that the penis might creep out from the pocket even if the movement of a wearer' s body is transmitted to the diaper. The core lying above the penis pocket ensures that the discharged urine is absorbed by the core from its lower surface and retained therein. The core functions as the barrier adapted to prevent the amount of urine discharged within the penis pocket from seeping in the upper covering section of the first sheet.

According to the embodiment in which the outer surface of the upper covering section of the first sheet except for the cleaved zone of the core is covered with the liquid-impervious third sheet, it is unlikely that any amount of urine might exude to the outer surface of the third sheet even if a certain amount of urine seep through the upper covering section of the first sheet. In this way, it is possible to reliably prevent any amount of urine from exuding to the inner side of the diaper.

## Claims

1. A disposable diaper having a longitudinal direction, a transverse direction, a front waist region, a rear waist region and a crotch region extending between said front and rear waist regions comprising:
a liquid-absorbent core having a body facing upper surface and a lower surface opposite to said upper surface and extending between said front and rear waist regions;
a liquid-pervious first sheet having an upper covering section adapted to cover said upper surface of said core and a lower covering section adapted to cover said lower surface of said core;
a liquid-impervious second sheet lying outside said lower covering section of said first sheet;
said core being provided with a cleaved zone extending in said longitudinal direction and extending through a thickness of said core, said upper covering section of said first sheet continuously extending downward from said upper surface of said core along said cleaved zone and further extending outward in said transverse direction so as to define a penis pocket extending in said longitudinal direction below said cleaved zone.

2. The diaper according to Claim 1, wherein said lower covering section of said first sheet extends downward along said cleaved zone of said core and further extends outward in said transverse direction so that said lower covering section cooperates with said upper covering section to define said penis pocket.

3. The diaper according to Claim 1 or 2, wherein said lower surface of said core is let free from said lower covering section of said first sheet and said lower covering section is joined to said second sheet only along a peripheral edge of said core so that said penis pocket is movable below said cleaved zone.

4. The diaper according to any one of Claims 1 through 3, wherein, except said cleaved zone of said core, said upper covering section of said first sheet has an outer surface thereof covered with a liquid-impervious third sheet.

5. The diaper according to any one of Claims 1 through 4, wherein, of said front and rear waist regions and said crotch region, at least said front waist region is formed in a transversely middle zone thereof with said cleaved zone of said core.
